# EUROPEAN PATENT APPLICATION

(11) **EP 4 666 958 A1**
(43) Date of publication of application: **24.12.2025**
(21) Application number: 24182994.4
(22) Date of filing: 19.06.2024
(51) Int. Cl.: A61B 8/08, A61B 6/00, A61B 6/50

(54) **DETERMINING VESSEL CROSS-SECTIONAL GEOMETRY**

(71) Applicant: Koninklijke Philips N.V., 5656 AG Eindhoven (NL)
(72) Inventor: OLIVAN BESCOS, Javier, Eindhoven (NL); HENDRIKS, Bernardus Hendrikus Wilhelmus, Eindhoven (NL); ADMIRAAL, Alexander Johannes, 5656AG Eindhoven (NL); GRANDIA, Leo, Eindhoven (NL); BACHVAROV, Chavdar Ludmilov, Eindhoven (NL); ELENBAAS, Thijs, Eindhoven (NL)
(74) Representative: Philips Intellectual Property & Standards

(57) **Abstract**

A system for determining a cross-sectional geometry of a vessel (110), is provided. The system includes one or more processors. The processor(s) receive intravascular imaging device data representing a cross sectional geometry (w₁, h₁) of the vessel (110). The processor(s) also receive fluoroscopic data (150). The fluoroscopic data includes one or more fluoroscopic images representing the intravascular imaging device (140) in the vessel (110) during the generation of the intravascular imaging device data (130). The fluoroscopic image(s) are registered to the angiographic image(s) to determine an orientation (α, β) of the intravascular imaging device (140) with respect to the vessel (110) in the angiographic image(s). The cross sectional geometry (w₁, h₁) of the vessel (110) is then adjusted based on the orientation (α, β) of the intravascular imaging device (140) with respect to the vessel (110) in the one or more angiographic images. The adjusted cross-sectional geometry (w'₁, h'₁) of the vessel (110) is then outputted.

## Description

### TECHNICAL FIELD

The present disclosure relates to determining a cross-sectional geometry of a vessel. A system, a computer-implemented method, and a computer program product, are disclosed.

### BACKGROUND

Medical investigations relating to the vasculature are often performed using intravascular imaging. For instance, percutaneous coronary intervention "PCI" procedures are often performed using intravascular ultrasound "IVUS" imaging. Optical Coherence Tomography "OCT", and Photoacoustic imaging, may likewise be used in such procedures. Intravascular imaging is used to provide detailed vessel information. For instance it may be used to determine vessel geometry, to investigate plaque burden, to investigate the presence of calcium, and so forth. The information obtained from intravascular imaging may be used for a variety of purposes, including assessing the need for a treatment procedure on the vessel, performing stent sizing, and so forth.

However, information relating to the cross-sectional geometry of vessels that is obtained from intravascular imaging devices can be misleading. This is due to intravascular imaging devices having a relatively smaller diameter than the diameter of vessels in which they are used, combined with the tortuous nature of vessels. The high degree of spatial freedom arising from the relatively smaller diameter of an intravascular imaging device can lead to a range of different measurements of the cross sectional geometry of a vessel. This, in-turn, impacts the quality of investigations that are performed using intravascular imaging devices.

Thus, there remains a need to improve the reliability of vessel cross-sectional geometry information that is obtained from intravascular imaging devices.

### SUMMARY

According to one aspect of the present disclosure, a system for determining a cross-sectional geometry of a vessel, is provided. The system includes one or more processors configured to:
receive intravascular imaging device data representing a cross sectional geometry of the vessel, wherein the intravascular image data is generated by an intravascular imaging device disposed in the vessel;
receive fluoroscopic data, the fluoroscopic data comprising one or more fluoroscopic images representing the intravascular imaging device in the vessel during the generation of the intravascular imaging device data;
receive angiographic data, the angiographic data comprising one or more angiographic images representing the vessel; and
for at least one of the one or more fluoroscopic images:
   register the fluoroscopic image to the one or more angiographic images to determine an orientation of the intravascular imaging device with respect to the vessel in the one or more angiographic images;
   adjust the cross sectional geometry of the vessel based on the orientation of the intravascular imaging device with respect to the vessel in the one or more angiographic images; and
   output the adjusted cross-sectional geometry of the vessel.

In the above system, the fluoroscopic image(s) represent the intravascular imaging device in the vessel during the generation of the intravascular imaging device data. The registration of the fluoroscopic image(s) to the angiographic image(s) is used to determine an orientation of the intravascular imaging device with respect to the vessel in the angiographic image(s) at the time at which the corresponding intravascular imaging device data is generated. A fluoroscopic image depicts the intravascular imaging device but may only poorly depict the vessel. An angiographic image depicts the vessel. The registration of the fluoroscopic image(s) to the angiographic image(s) therefore provides an accurate orientation of the intravascular device with respect to the vessel. The cross sectional geometry of the vessel, as represented in the intravascular imaging device data, is then adjusted based on the orientation of the intravascular imaging device with respect to the vessel in the angiographic image(s). In so doing, an accurate adjustment of the cross sectional geometry of the vessel is provided. Thus, the system improves the reliability of cross-sectional geometry information that is obtained from the intravascular imaging device.

Further aspects, features, and advantages of the present disclosure will become apparent from the following description of examples, which is made with reference to the accompanying drawings.

### BRIEF DESCRIPTION OF THE DRAWINGS

Fig. 1 is a schematic diagram illustrating an example of a system 100 for determining a cross-sectional geometry of a vessel 110, in accordance with some aspects of the present disclosure.
Fig. 2 is a flowchart illustrating an example of a computer-implemented method of determining a cross-sectional geometry of a vessel, in accordance with some aspects of the present disclosure.
Fig. 3 is a schematic diagram illustrating examples of cross-sectional geometries of a vessel 110, in accordance with some aspects of the present disclosure.

### DETAILED DESCRIPTION

Examples of the present disclosure are provided with reference to the following description and Figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a system, may be implemented in a computer-implemented method, and in a computer program product, in a corresponding manner.

In the following description, reference is made to examples of a system for determining a cross-sectional geometry of a vessel. In some examples, reference is made to a vessel in the form of a coronary artery. It is, however, to be appreciated that the artery, and the heart, serve only as examples. In general, the system may be used to determine a cross-sectional geometry of any type of blood vessel, and the blood vessel may be in any anatomical region. For instance, the system may be used to determine a cross-sectional geometry of an artery, or a vein, and the blood vessel may be in the heart, the brain, or in a peripheral region such as the leg, the kidney, and so forth.

It is noted that the computer-implemented methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, a peer-to-peer architecture, the Internet, or the Cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid-state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray^{™} and DVD.

It is also noted that some operations that are described as being performed by the one or more processors of a system disclosed herein may be implemented using artificial intelligence techniques. Suitable techniques may include machine learning techniques, deep learning techniques, and neural networks. For instance, one or more neural networks, may be trained in a supervised, or in some cases unsupervised, manner, to implement the operations that are performed by the one or more processors.

As mentioned above, there remains a need to improve the reliability of vessel cross-sectional geometry information that is obtained from intravascular imaging devices.

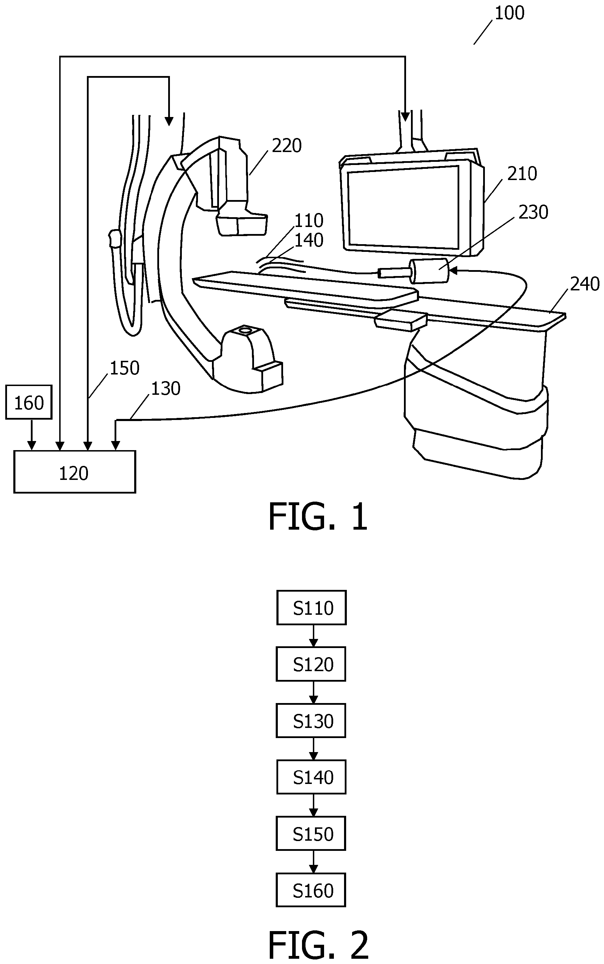

Fig. 1 is a schematic diagram illustrating an example of a system 100 for determining a cross-sectional geometry of a vessel 110, in accordance with some aspects of the present disclosure. Fig. 2 is a flowchart illustrating an example of a computer-implemented method of determining a cross-sectional geometry of a vessel, in accordance with some aspects of the present disclosure. It is noted that operations that are described as being performed by the one or more processors 120 of the system 100 illustrated in Fig. 1, may also be performed in the method illustrated in Fig. 2. Likewise, operations that are described in relation to the method described with reference to Fig. 2 may also be performed by the one or more processors 120 of the system 100 illustrated in Fig. 1. With reference to Fig. 1, and Fig. 2, the system 100 for determining a cross-sectional geometry of a vessel 110, includes one or more processors 120 configured to:
receive S110 intravascular imaging device data 130 representing a cross sectional geometry w₁, h₁ of the vessel 110, wherein the intravascular image data 130 is generated by an intravascular imaging device 140 disposed in the vessel 110;
receive S120 fluoroscopic data 150, the fluoroscopic data comprising one or more fluoroscopic images representing the intravascular imaging device 140 in the vessel 110 during the generation of the intravascular imaging device data 130;
receive S130 angiographic data 160, the angiographic data comprising one or more angiographic images representing the vessel 110; and
for at least one of the one or more fluoroscopic images:
   register S140 the fluoroscopic image to the one or more angiographic images to determine an orientation α, β of the intravascular imaging device 140 with respect to the vessel 110 in the one or more angiographic images;
   adjust S150 the cross sectional geometry w₁, h₁ of the vessel 110 based on the orientation α, β of the intravascular imaging device 140 with respect to the vessel 110 in the one or more angiographic images; and
   output S160 the adjusted cross-sectional geometry w'₁, h'₁ of the vessel 110.

In the above system 100, the fluoroscopic image(s) represent the intravascular imaging device in the vessel during the generation of the intravascular imaging device data. The registration of the fluoroscopic image(s) to the angiographic image(s) is used to determine an orientation of the intravascular imaging device with respect to the vessel in the angiographic image(s) at the time at which the corresponding intravascular imaging device data is generated. A fluoroscopic image depicts the intravascular imaging device but may only poorly depict the vessel. An angiographic image depicts the vessel. The registration of the fluoroscopic image(s) to the angiographic image(s) therefore provides an accurate orientation of the intravascular device with respect to the vessel. The cross sectional geometry of the vessel, as represented in the intravascular imaging device data, is then adjusted based on the orientation of the intravascular imaging device with respect to the vessel in the angiographic image(s). In so doing, an accurate adjustment of the cross sectional geometry of the vessel is provided. Thus, the system 100 improves the reliability of cross-sectional geometry information that is obtained from the intravascular imaging device.

The operations that are performed by the processor(s) of the system 100 are described in more detail below.

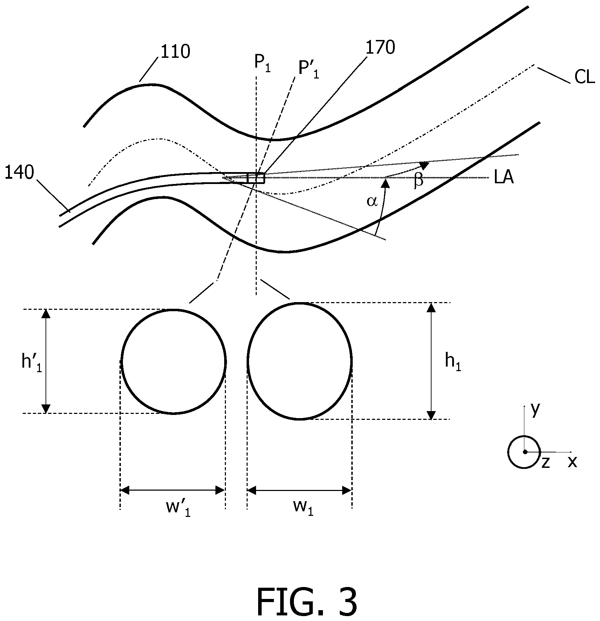

Referring initially to the operation S110 illustrated in Fig. 2; in this operation, intravascular imaging device data 130 is received. The intravascular imaging device data 130 may generated by various types of intravascular imaging devices. For instance, the intravascular imaging device data 130 may be generated by an IVUS imaging device, or an OCT imaging device, or a photoacoustic imaging device. The intravascular imaging device data 130 that is received in the operation S110 represents a cross sectional geometry w₁, h₁ of the vessel 110. The intravascular imaging device data 130 may represent a cross sectional geometry of the vessel such as a cross-sectional shape of the vessel, or a cross-sectional dimension of the vessel. Examples of cross-sectional dimensions of the vessel include a cross-sectional diameter of the vessel, or a cross-sectional area of the vessel. The intravascular imaging device data 130 may represent a dimension, or a shape, of the cross sectional geometry of the vessel lumen, or the vessel wall, or any deposits that may be within the vessel, for example. With reference to the example illustrated in Fig. 3, the intravascular imaging device data 130 may represent a maximum dimension of the vessel lumen in each of two orthogonal axes, x, and y, as illustrated in Fig. 3 via the parameters h₁ and w₁.

By way of an example, IVUS data includes a distribution of pixel values representing an intensity of reflected ultrasound signals across a plane that transversely intersects a vessel. The pixel values correspond to the locations of vessel features such as the vessel lumen, the vessel wall, (calcium) deposits within the vessel, and so forth. The pixel values therefore provide information representing the cross-sectional geometry of the vessel.

The intravascular image data 130 that is received in the operation S110 is generated by an intravascular imaging device 140 disposed in the vessel 110. As mentioned above, the intravascular imaging device 140 may be disposed in various types of blood vessels. In general, the vessel 110 may be any type of blood vessel, and the blood vessel may be in any anatomical region. By way of an example, the vessel 110 may be a coronary artery.

The intravascular image data 130 that is received in the operation S110 may be received from various sources. For example, the intravascular image data 130 may be received from an intravascular imaging device 140 such as one of the intravascular imaging devices described above. The intravascular image data 130 may alternatively be received from a different source, such as from a computer-readable storage medium, of from the Internet, or the Cloud, and so forth. The intravascular imaging device data 130 may be received via any form of data communication in the operation S110, including via wired, or wireless, or optical fiber communication. By way of some examples, when wired data communication is used, the communication may take place via electrical signals that are transmitted on an electrical cable. When wireless data communication is used, the communication may take place via RF or infrared signals. When an optical fiber data communication is used, the communication takes place via optical signals that are transmitted on an optical fiber.

Referring now to the operation S120 illustrated in Fig. 2; in this operation, fluoroscopic data 150, is received. The fluoroscopic data 150 may generated by various types of fluoroscopic imaging systems. A fluoroscopic imaging system generally refers to an imaging device that uses X-rays to generate a temporal sequence of images. An X-ray dose that is used to acquire a fluoroscopic image may be relatively lower than a dose used to acquire an angiographic image. The fluoroscopic imaging system may be a projection X-ray imaging system. An example of a fluoroscopic imaging system that may be used to generate the fluoroscopic data 150 is the Azurion 5 system marketed by Philips Healthcare, Best, The Netherlands.

The fluoroscopic data 150 that is received in the operation S120 comprises one or more fluoroscopic images representing the intravascular imaging device 140 in the vessel 110 during the generation of the intravascular imaging device data 130. Since the fluoroscopic image(s) represent the intravascular imaging device 140 in the vessel 110 during the generation of the intravascular imaging device data 130, the fluoroscopic image(s) capture the position of the intravascular imaging device 140 in the vessel at the time at which the intravascular imaging device data 130 is generated. The fluoroscopic image(s) typically capture the shape of the intravascular imaging device along a portion of its longitudinal axis. Thus, the fluoroscopic image(s) represent the orientation of the intravascular imaging device data 130. The fluoroscopic image(s) represent the intravascular imaging device 140 by virtue of the X-ray attenuation of the intravascular imaging device 140. IVUS imaging devices typically include materials such as polymers and metals having X-ray attenuation coefficients that render portions of the IVUS imaging device visible in fluoroscopic images. The intravascular imaging device 140 may include one or more fiducial markers that similarly serve to enhance the visibility of portions of the intravascular imaging device 140 in fluoroscopic images. As mentioned above, the fluoroscopic imaging system may be a projection X-ray imaging system. In this case, the fluoroscopic data 150 that is received in the operation S 120 may include two-dimensional fluoroscopic image(s) representing the intravascular imaging device 140.

The fluoroscopic data 150 that is received in the operation S 120 may be received from various sources. For example, the fluoroscopic data 150 may be received from a fluoroscopic imaging system, as described above. The fluoroscopic data 150 may alternatively be received from a different source, such as from a computer-readable storage medium, of from the Internet, or the Cloud, and so forth. The fluoroscopic data 150 that is received in the operation S 120 may be received via any form of data communication in the operation S 120, as described above in relation to the intravascular imaging device data 130.

Referring now to the operation S 130 illustrated in Fig. 2; in this operation, angiographic data 160 is received. The angiographic data comprises one or more angiographic images representing the vessel 110.

The angiographic data 160 that is received in the operation S 130 may be generated by various types of medical imaging systems. These include two-dimensional "2D" medical imaging systems such as projection X-ray imaging systems and 2D ultrasound imaging systems, and also three-dimensional, i.e. "volumetric" medical imaging systems such as computed tomography "CT" imaging systems, magnetic resonance imaging "MRI" systems, 3D ultrasound imaging systems, positron emission tomography "PET" imaging systems, and single photon emission computed tomography "SPECT" imaging systems. In examples in which the angiographic data is generated by a projection X-ray imaging system, or by a CT imaging system, the imaging system may be a spectral X-ray projection imaging system, or a spectral CT imaging system, respectively. Such imaging systems generate angiographic data representing X-ray attenuation within multiple different energy intervals. The angiographic data generated by such imaging systems may be processed, e.g. using various material decomposition algorithms, in order to distinguish between media that have similar X-ray attenuation values when measured within a single energy interval, and which would be indistinguishable in angiographic data generated by conventional projection X-ray imaging systems, or conventional CT imaging systems. Thus, angiographic data generated by spectral X-ray projection imaging systems, or by spectral CT imaging systems, may be processed to provide angiographic images with improved specificity to materials such as contrast agent, tissue, bone, the constituents of plaque, and so forth.

With continued reference to the operation S130; in some examples, the angiographic data 160 is generated subsequent to the injection of a contrast agent into the vasculature of a subject. The contrast agent serves to improve the contrast between blood and surrounding tissue in the angiographic image(s). In these examples, the angiographic image(s) may be referred-to as contrast-enhanced angiographic image(s). Contrast-enhanced extracorporeal images may be generated by a projection X-ray imaging system, or by a CT imaging system, or by an MRI system. Extracorporeal MRI images may alternatively be generated in the absence of a contrast agent, and are often referred-to as non-contrast-enhanced MR angiography "NC-MRA" images.

With continued reference to the operation S130; the angiographic data 160 comprises one or more angiographic images representing the vessel 110. The angiographic image(s) may also represent other vessel in addition to the vessel 110. For instance, the angiographic image(s) may represent a portion of the coronary tree that includes the vessel 110.

With continued reference to the operation S130; in one example, the angiographic data 160 is generated prior to performing an interventional procedure on the vessel 110. In this example, the angiographic image(s) may be referred-to as a pre-interventional procedure angiographic image(s). The angiographic data 160 may have been generated some seconds, minutes, hours, or even days, or a longer period, before performing the interventional procedure on the vessel 120. In another example, the angiographic data is generated during an interventional procedure on the vessel 110. In this example, the angiographic image(s) may be referred-to as intra-interventional procedure angiographic image(s).

With continued reference to the operation S130; the angiographic data 160 may be received from various sources in the operation S130. For instance, the angiographic data may be received from a medical imaging system such as one of the medical imaging systems described above. Alternatively, the angiographic data 160 may be received from a different source, such as from a computer readable storage medium, or from the Internet, or from the Cloud, and so forth. In general, the angiographic data 160 may be received via any form of data communication in the operation S130, as described above in relation to the intravascular imaging device data 130.

Referring now to the operation S140 illustrated in Fig. 2; in this operation the fluoroscopic image(s) are registered to the angiographic image(s) to determine an orientation α, β of the intravascular imaging device 140 with respect to the vessel 110 in the one or more angiographic images.

In general, the operation of registering S140 the fluoroscopic image(s) to the angiographic images, may be performed based on a shape of the intravascular imaging device 140) in the fluoroscopic image(s) and a shape of the vessel 110 in the one or more angiographic images. Thus, the correspondence between the shape of the intravascular imaging device 140 in the fluoroscopic image(s), and the shape of the vessel 110 in the one or more angiographic images, is used to register the images. As mentioned above, a fluoroscopic image depicts the intravascular imaging device but may only poorly depict the vessel. The angiographic image(s) depict the vessel. The result of the operation S140 is to provide the position, and also the orientation, of the intravascular imaging device 140 with respect to the vessel 110 in the angiographic image(s). Various registration techniques may be used to perform the registration in the operation S140. An example of a registration technique that may be used perform this registration in a cardiac vessel undergoing cardiac motion is described in the document WO 2012/107857 A1.

The orientation that is determined in the operation S140 may be defined in various ways depending on whether the angiographic image(s) are volumetric angiographic images, or projection angiographic images. For instance, if the angiographic data 160 includes a volumetric angiographic image representing the vessel 110, then projection fluoroscopic images representing the vessel may be registered to the volumetric angiographic image to determine an orientation of the intravascular imaging device 140 with respect to the vessel in the angiographic image in a three-dimensional coordinate system. The orientation may be defined in a Cartesian, or a Polar coordinate system, for example. If instead, the angiographic data 160 includes only a single projection angiographic image representing the vessel 110, then projection fluoroscopic images representing the vessel may be registered to the projection angiographic image to determine an orientation of the intravascular imaging device 140 with respect to the vessel in the angiographic image in a two-dimensional coordinate system.

In one example, the fluoroscopic data 150 that is received in the operation S120 comprises one or more fluoroscopic projection images, and the angiographic data 160 comprises the one or more projection angiographic images. The one or more fluoroscopic projection images, and the one or more projection angiographic images represent projections of the vessel 110 from a same viewing angle with respect to the vessel.

In this example, using the same viewing angle to generate the fluoroscopic and angiographic images facilitates the registration between the fluoroscopic image(s) and the projection angiographic image(s).

In another example, the angiographic data 160 includes multiple projection angiographic images that represent the vessel from different viewing angles. In this example, projection fluoroscopic images that represent the vessel 110 from a same viewing angle with respect to the vessel as at least one projection angiographic image may be registered to the corresponding projection angiographic image to determine an orientation of the intravascular imaging device 140 with respect to the vessel in that angiographic image. The correspondence between the angiographic projection images inherently provides an orientation of the intravascular imaging device 140 with respect to the vessel in the other angiographic image. Thus, projection fluoroscopic images may be used in combination with multiple projection angiographic images that represent the vessel from different viewing angles, to determine an approximation to an orientation of the intravascular imaging device 140 with respect to the vessel in a three-dimensional coordinate system.

If volumetric fluoroscopic image(s) received are received in the operation S120, or if projection fluoroscopic images representing the vessel from different viewing angles are received in the operation S120, then these types of images may be registered to angiographic image(s) in the operation S140 to obtain a more accurate orientation in the examples described above.

In the examples described above the orientation that is determined in the operation S140 may include an angle in a two-dimensional coordinate system, or an angle in a three-dimensional coordinate system. The coordinate system may be a coordinate system of the angiographic image(s). The orientation that is determined in the operation S140 may alternatively or additionally include a rotational angle. A rotational angle may be determined based on a projected shape of a feature of the intravascular imaging device. For instance, if the intravascular imaging device is provided with a fiducial marker, or a pattern of fiducial markers, that has a projected shape that varies in accordance with its rotational angle, then its rotational angle may be determined in a projection fluoroscopic image. Alternatively, if the intravascular imaging device has an asymmetrical shape, then a rotational angle of the intravascular imaging device may be determined from the projected shape of the intravascular imaging device in projection fluoroscopic images representing the intravascular imaging device from different viewing angles.

The orientation that is determined in the operation S140 may be determined with respect to various references in the angiographic image(s). In some examples, the orientation of the intravascular imaging device 140 is determined with respect to a centerline, CL, of the vessel 110 in the angiographic image(s). The centerline of the vessel can be accurately defined, and so it may serve as a reliable reference. Instead of using the centerline, another reference in the vessel may be used, such as a vessel wall, a branch of the vessel, a bifurcation in the vessel, and so forth.

Referring now to the operation S150 illustrated in Fig. 2. In this operation, the cross sectional geometry w₁, h₁ of the vessel 110 is adjusted based on the orientation α, β of the intravascular imaging device 140 with respect to the vessel 110 in the one or more angiographic images. An example of this operation is described with reference to Fig. 3, which is a schematic diagram illustrating examples of cross-sectional geometries of a vessel 110, in accordance with some aspects of the present disclosure. The upper portion of Fig. 3 illustrates an intravascular imaging device 140 within a vessel 110. An imaging portion 170 of the intravascular imaging device is arranged towards a distal end of the intravascular imaging device 140. A centerline of the vessel 110 is labelled CL. During its use, the intravascular imaging device 140 generates intravascular image data representing a cross sectional geometry of the vessel 110. In the illustrated example, the intravascular imaging device 140 generates intravascular image data representing a cross sectional geometry of the vessel 110 in the plane P₁. The plane P₁ is arranged normally with respect to a longitudinal axis LA of the intravascular imaging device 140. The intravascular imaging device 140 illustrated in Fig. 3 may be an IVUS imaging device, for example.

In the example illustrated in Fig. 3, the actual cross-sectional shape of the vessel is a circle. However, due to the relatively smaller diameter of the intravascular imaging device 140 in relation to the diameter of the vessel 110, the imaging portion 170 of the intravascular imaging device has a high degree of spatial freedom within the vessel. As a result of this spatial freedom, a range of different cross sectional geometries may be measured by the intravascular imaging device 140. In the example illustrated in Fig. 3, the longitudinal axis LA of the intravascular imaging device 140 is arranged obliquely at an angle α with respect to the centerline CL of the vessel. As a result, the measured shape of the cross section of the vessel is deformed from its actual circular shape and is instead measured with an oval shape in the plane P₁, as shown in the lower right-hand portion of Fig. 3. This can be misleading since the actual cross-sectional shape of the vessel 110 is a circle.

In order to compensate for the spatial freedom described above, in the operation S150 the cross sectional geometry w₁, h₁ of the vessel 110 is adjusted based on the orientation α, β of the intravascular imaging device 140 with respect to the vessel 110 in the one or more angiographic images. Thus, the orientation α, β of the intravascular imaging device 140 with respect to the vessel 110 in the one or more angiographic images, and which was obtained in the operation S140 described above, is used to adjust the cross sectional geometry w₁, h₁ of the vessel 110.

In general, the adjusting that is performed in the operation S150 may be performed in a two-dimensional space, or in a three-dimensional space, depending on whether the orientation is determined in a two-dimensional coordinate system, or in a three-dimensional coordinate system, in the operation S140. For instance, if a single projection angiographic image is received in the operation S130, then the orientation of the intravascular imaging device 140, and consequently the adjustment, may be made in a two-dimensional coordinate system. With reference to the example illustrated in Fig. 3, if the angiographic image(s) received in the operation S130 includes a projection angiographic image corresponding to the x, y, plane of the drawing, then the angle α may be measured. The angle α represents the orientation of the intravascular imaging device 140 with respect to the centerline CL of the vessel in the plane of the angiographic image. If instead, the angiographic image(s) received in the operation S130 includes an volumetric angiographic image, then the orientation of the intravascular imaging device 140, and consequently the adjustment, may be made in a three-dimensional coordinate system. With reference to the example illustrated in Fig. 3, if the angiographic image(s) received in the operation S130 includes a volumetric angiographic image, then the angle β may be measured in addition to the angle α. The angle β represents the orientation of the intravascular imaging device 140 with respect to the centerline in the x, z, plane, i.e. an out-of-plane direction with respect to the plane of the drawing.

In some examples, the centerline CL of the vessel 110 is used a reference with which to perform the adjusting in the operation S150. As mentioned above, the centerline of the vessel can be accurately defined, and so it may serve as a reliable reference. For instance, in one example, the operation of registering S140 the fluoroscopic image to the one or more angiographic images to determine an orientation α, β of the intravascular imaging device 140 with respect to the vessel 110 in the one or more angiographic images, comprises determining the orientation of the intravascular imaging device 140 with respect to a centerline, CL, of the vessel 110 in the one or more angiographic images. In this example, the operation of adjusting S150 the cross sectional geometry w₁, h₁ of the vessel 110 based on the orientation α, β of the intravascular imaging device 140 with respect to the vessel 110 in the one or more angiographic images, is then performed based on the orientation α, β of the intravascular imaging device 140 with respect to the centerline, CL, of the vessel 110 in the one or more angiographic images.

The adjusted cross sectional geometry w₁, h₁ of the vessel 110 that is generated in the operation S150 may be obtained using known geometric calculations. These may be applied to the dimension, or to the shape, of the vessel, for example. For example, geometric calculations may be used to scale dimensions w₁ and/or h₁ of the vessel 110 illustrated in Fig. 3 and to thereby provide the adjusted dimensions w₁ and/or h₁, respectively. Geometric calculations may similarly be used to deform the shape of a vessel in each of two orthogonal directions in order to provide a vessel with an adjusted cross-sectional shape.

In one example, the operation of adjusting S150 the cross sectional geometry w₁, h₁ of the vessel 110 based on the orientation α, β, of the intravascular imaging device 140 with respect to the vessel 110 in the one or more angiographic images, comprises providing an adjusted cross-sectional geometry w'₁, h'₁ of the vessel 110 in a virtual plane arranged perpendicularly, or normally, with respect to a centerline, CL, of the vessel 110.

As mentioned above, the centerline of the vessel provides a reliable reference. Moreover the cross-sectional geometry in a virtual plane that is arranged perpendicularly, or normally, with respect to a centerline, CL, of the vessel 110, may be considered to represent the actual shape of the vessel. The adjusted cross-sectional geometry w'₁, h'₁ of the vessel 110 in the virtual plane may be provided by deforming the vessel using geometric calculations, as described above.

Referring now to the operation S160 illustrated in Fig. 2; in this operation, the adjusted cross-sectional geometry w'₁, h'₁ of the vessel 110 is outputted.

The adjusted cross-sectional geometry may be outputted in various ways in the operation S160. For instance, the adjusted cross-sectional geometry may be outputted to a display device, such as to the display 210 illustrated in Fig. 1, or to a virtual/ augmented reality display device, or to printer, or to a computer-readable storage medium, and so forth.

Further examples of the system 100 are described in the examples below.

In one example an intravascular image is outputted. In this example, the intravascular imaging device data 130 comprises an intravascular image representing the cross sectional geometry w₁, h₁ of the vessel 110. In this example, the operation of adjusting S150 the cross sectional geometry w₁, h₁ of the vessel 110 comprises adjusting the cross sectional geometry of the vessel in the intravascular image based on the orientation α, β of the intravascular imaging device 140 with respect to the vessel 110 in the one or more angiographic images. The outputting S160 the adjusted cross-sectional geometry w'₁, h'₁ of the vessel 110, comprises outputting the intravascular image comprising the vessel with the adjusted cross sectional geometry w'₁, h'₁.

By providing an intravascular image that includes the vessel with the adjusted cross sectional geometry w'₁, h'₁, the system 100 informs a physician of the actual shape of the vessel. The intravascular image comprising the vessel with the adjusted cross sectional geometry w'₁, h'₁ may be obtained by using the orientation α, β, to deform the intravascular image representing the cross sectional geometry w₁, h₁ of the vessel 110.

In one example, the one or more processors 120 are configured to output an intravascular image comprising an original cross sectional geometry w₁, h₁ of the vessel 110. The intravascular image comprising the original cross sectional geometry of the vessel, is generated from the intravascular image representing the cross sectional geometry of the vessel.

By outputting the intravascular image with the original cross sectional geometry of the vessel, the system informs a physician of the deformation that is introduced via the orientation of the intravascular imaging device. The intravascular image comprising the original cross sectional geometry w₁, h₁ of the vessel 110, and the intravascular image comprising the vessel with the adjusted cross sectional geometry w'₁, h'₁, may be displayed side-by-side, for example.

In one example, the orientation α, β of the intravascular imaging device 140 with respect to the vessel 110 in the angiographic image is used to compute an angiographic image representing the adjusted cross sectional geometry w'₁, h'₁ of the vessel 110. In this example, the angiographic data 160 comprises a volumetric angiographic image representing the vessel 110, or a plurality of projection angiographic images representing projections of the vessel from different viewing angles with respect to the vessel. The one or more processors 120 are configured to compute, and to output, an angiographic image representing the adjusted cross sectional geometry w'₁, h'₁ of the vessel 110. The angiographic image representing the adjusted cross sectional geometry w'₁, h'₁ of the vessel 110 is generated from the volumetric angiographic image, or the plurality of projection angiographic images, respectively, based on the orientation α, β of the intravascular imaging device 140 with respect to the vessel 110 in the angiographic image, or the plurality of projection angiographic images, respectively.

The angiographic image representing the adjusted cross sectional geometry w'₁, h'₁ of the vessel 110 may be obtained by generating a view from the angiographic data that corresponds to the adjusted cross sectional geometry (w₁, h₁) of the vessel (110). The angiographic image representing the adjusted cross sectional geometry w'₁, h'₁ of the vessel 110 may provide additional information that facilitates interpretation of the adjusted cross-sectional geometry w'₁, h'₁ of the vessel 110. For instance, the angiographic image may show vessel features such as calcium deposits which explain the vessel's adjusted cross sectional geometry.

In one example, the received intravascular imaging device data 130 represents a cross sectional geometry of the vessel 110 at each of a plurality of positions along the vessel 110. The received fluoroscopic data 150 represents the intravascular imaging device 140 in the vessel 110 during the generation of the intravascular imaging device data 130 at the positions. In this example, the one or more processors 120 are configured to perform the registering, and the adjusting, and the outputting, for each of a plurality of the fluoroscopic images using the fluoroscopic images corresponding to the positions to provide the adjusted cross-sectional geometry of the vessel 110 at a plurality of the positions along the vessel.

In this example, the positions may correspond to the positions of the intravascular imaging device during a so-called pullback procedure. By providing the adjusted cross-sectional geometry of the vessel 110 at the plurality of the positions along the vessel, the system informs a physician of the actual shape of the vessel along its length.

In a related example, interpolation is used to determine a cross-sectional geometry of the vessel 110 for the one or more further positions along the vessel. In this example, the intravascular imaging device data 130 represents a cross sectional geometry of the vessel 110 at one or more further positions along the vessel. The one or more processors 120 are configured to:
generate, based on the adjusted cross-sectional geometry of the vessel 110 at one or more of the positions along the vessel, an interpolated cross-sectional geometry of the vessel 110 for the one or more further positions along the vessel; and
output the interpolated cross-sectional geometry of the vessel 110 for the one or more further positions along the vessel.

The interpolated shape of the vessel may be used to compensate for coarsely-sampled, or missing data.

In one example, the one or more processors 120 are configured to output an alarm signal if the orientation α, β of the intravascular imaging device 140 with respect to the vessel 110 meets a threshold condition.

The alarm may alert a physician to the risk of mis-interpreting an un-adjusted cross sectional geometry w₁, h₁ of the vessel 110. In this example, the alarm may be generated if the magnitude of the angular deviation between the longitudinal axis of the intravascular imaging device 140 and the centerline of the vessel exceeds a predetermined value, for example.

It is noted that in addition to the one or more processors 120, the system 100 may also include one or more of: a fluoroscopic imaging system for providing the angiographic image data, such as for example the projection X-ray imaging system 220 illustrated in Fig. 1; an intravascular imaging device controller 230; a display device, such as the monitor 230 illustrated in Fig. 1, for displaying the adjusted cross-sectional geometry w'₁, h'₁ of the vessel 110, the fluoroscopic image(s), the angiographic image(s), other outputs generated by the one or more processors 120, and so forth; a patient bed 240; and a user input device (not illustrated in Fig. 1) configured to receive user input in relation to the operations performed by the one or more processors 120, such as a keyboard, a mouse, a touchscreen, and so forth.

In another example, a computer-implemented method of determining a cross-sectional geometry of a vessel 110, is provided. The method comprises:
receiving S110 intravascular imaging device data 130 representing a cross sectional geometry w₁, h₁ of the vessel 110, wherein the intravascular image data 130 is generated by an intravascular imaging device 140 disposed in the vessel 110;
receiving S120 fluoroscopic data 150, the fluoroscopic data comprising one or more fluoroscopic images representing the intravascular imaging device 140 in the vessel 110 during the generation of the intravascular imaging device data 130;
receiving S130 angiographic data 160, the angiographic data comprising one or more angiographic images representing the vessel 110; and
for at least one of the one or more fluoroscopic images:
   registering S140 the fluoroscopic image to the one or more angiographic images to determine an orientation α, β of the intravascular imaging device 140 with respect to the vessel 110 in the one or more angiographic images;
   adjusting S150 the cross sectional geometry w₁, h₁ of the vessel 110 based on the orientation α, β of the intravascular imaging device 140 with respect to the vessel 110 in the one or more angiographic images; and
   outputting S160 the adjusted cross-sectional geometry w'₁, h'₁ of the vessel 110.

In another example, a computer program product, is provided. The computer program product comprises instructions which when executed by one or more processors 120, cause the one or more processors to carry out a method of determining a cross-sectional geometry of a vessel 110. The method comprises:
receiving S110 intravascular imaging device data 130 representing a cross sectional geometry w₁, h₁ of the vessel 110, wherein the intravascular image data 130 is generated by an intravascular imaging device 140 disposed in the vessel 110;
receiving S120 fluoroscopic data 150, the fluoroscopic data comprising one or more fluoroscopic images representing the intravascular imaging device 140 in the vessel 110 during the generation of the intravascular imaging device data 130;
receiving S130 angiographic data 160, the angiographic data comprising one or more angiographic images representing the vessel 110; and
for at least one of the one or more fluoroscopic images:
   registering S140 the fluoroscopic image to the one or more angiographic images to determine an orientation α, β of the intravascular imaging device 140 with respect to the vessel 110 in the one or more angiographic images;
   adjusting S150 the cross sectional geometry w₁, h₁ of the vessel 110 based on the orientation α, β of the intravascular imaging device 140 with respect to the vessel 110 in the one or more angiographic images; and
   outputting S160 the adjusted cross-sectional geometry w'₁, h'₁ of the vessel 110.

The above examples are to be understood as illustrative of the present disclosure, and not restrictive. Further examples are also contemplated. For instance, the examples described in relation to a system, may also be provided by the computer-implemented method, or by the computer program product, or by the computer-readable storage medium, in a corresponding manner. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

## Claims

1. A system (100) for determining a cross-sectional geometry of a vessel (110), the system comprising one or more processors (120) configured to:
receive (S 110) intravascular imaging device data (130) representing a cross sectional geometry (w₁, h₁) of the vessel (110), wherein the intravascular image data (130) is generated by an intravascular imaging device (140) disposed in the vessel (110);
receive (S120) fluoroscopic data (150), the fluoroscopic data comprising one or more fluoroscopic images representing the intravascular imaging device (140) in the vessel (110) during the generation of the intravascular imaging device data (130);
receive (S130) angiographic data (160), the angiographic data comprising one or more angiographic images representing the vessel (110); and
for at least one of the one or more fluoroscopic images:
register (S140) the fluoroscopic image to the one or more angiographic images to determine an orientation (α, β) of the intravascular imaging device (140) with respect to the vessel (110) in the one or more angiographic images;
adjust (S150) the cross sectional geometry (w₁, h₁) of the vessel (110) based on the orientation (α, β) of the intravascular imaging device (140) with respect to the vessel (110) in the one or more angiographic images; and
output (S160) the adjusted cross-sectional geometry (w'₁, h'₁) of the vessel (110).

2. The system according to claim 1, wherein the registering (S140) the fluoroscopic image to the one or more angiographic images to determine an orientation (α, β) of the intravascular imaging device (140) with respect to the vessel (110) in the one or more angiographic images, comprises determining the orientation of the intravascular imaging device (140) with respect to a centerline (CL) of the vessel (110) in the one or more angiographic images; and
wherein the adjusting (S150) the cross sectional geometry (w₁, h₁) of the vessel (110) based on the orientation (α, β) of the intravascular imaging device (140) with respect to the vessel (110) in the one or more angiographic images, is performed based on the orientation (α, β) of the intravascular imaging device (140) with respect to the centerline (CL) of the vessel (110) in the one or more angiographic images.

3. The system according to claim 1 or claim 2, wherein the adjusting (S150) the cross sectional geometry (w₁, h₁) of the vessel (110) based on the orientation (α, β) of the intravascular imaging device (140) with respect to the vessel (110) in the one or more angiographic images, comprises providing an adjusted cross-sectional geometry (w'₁, h'₁) of the vessel (110) in a virtual plane arranged perpendicularly, or normally, with respect to a centerline (CL) of the vessel (110).

4. The system according to any previous claim, wherein the received intravascular imaging device data (130) represents a cross sectional geometry of the vessel (110) at each of a plurality of positions along the vessel (110) and wherein the received fluoroscopic data (150) represents the intravascular imaging device (140) in the vessel (110) during the generation of the intravascular imaging device data (130) at the positions; and
wherein the one or more processors (120) are configured to perform the registering, and the adjusting, and the outputting, for each of a plurality of the fluoroscopic images using the fluoroscopic images corresponding to the positions to provide the adjusted cross-sectional geometry of the vessel (110) at a plurality of the positions along the vessel.

5. The system according to claim 4, wherein the intravascular imaging device data (130) represents a cross sectional geometry of the vessel (110) at one or more further positions along the vessel; and
wherein the one or more processors (120) are further configured to:
generate, based on the adjusted cross-sectional geometry of the vessel (110) at one or more of the positions along the vessel, an interpolated cross-sectional geometry of the vessel (110) for the one or more further positions along the vessel; and
output the interpolated cross-sectional geometry of the vessel (110) for the one or more further positions along the vessel.

6. The system according to any previous claim, wherein the intravascular imaging device data (130) comprises an intravascular image representing the cross sectional geometry (w₁, h₁) of the vessel (110); and
wherein the adjusting (S150) the cross sectional geometry (w₁, h₁) of the vessel (110) comprises adjusting the cross sectional geometry of the vessel in the intravascular image based on the orientation (α, β) of the intravascular imaging device (140) with respect to the vessel (110) in the one or more angiographic images; and
wherein the outputting (S160) the adjusted cross-sectional geometry (w'₁, h'₁) of the vessel (110), comprises outputting the intravascular image comprising the vessel with the adjusted cross sectional geometry (w'₁, h'₁).

7. The system according to claim 6, wherein the one or more processors (120) are further configured to:
output an intravascular image comprising an original cross sectional geometry (w₁, h₁) of the vessel (110), wherein the intravascular image comprising the original cross sectional geometry of the vessel, is generated from the intravascular image representing the cross sectional geometry of the vessel.

8. The system according to any previous claim, wherein the angiographic data (160) comprises a volumetric angiographic image representing the vessel (110), or a plurality of projection angiographic images representing projections of the vessel from different viewing angles with respect to the vessel; and
wherein the one or more processors (120) are configured to compute, and to output, an angiographic image representing the adjusted cross sectional geometry (w'₁, h'₁) of the vessel (110); and
wherein the angiographic image representing the adjusted cross sectional geometry (w'₁, h'₁) of the vessel (110) is generated from the volumetric angiographic image, or the plurality of projection angiographic images, respectively, based on the orientation (α, β) of the intravascular imaging device (140) with respect to the vessel (110) in the angiographic image, or the plurality of projection angiographic images, respectively.

9. The system according to any previous claim, wherein the angiographic data (160) comprises a volumetric angiographic image representing the vessel (110).

10. The system according to any one of claims 1 - 8, wherein the fluoroscopic data (150) comprises one or more fluoroscopic projection images, and wherein the angiographic data (160) comprises the one or more projection angiographic images, and wherein the one or more fluoroscopic projection images, and the one or more projection angiographic images represent projections of the vessel (110) from a same viewing angle with respect to the vessel.

11. The system according to any previous claim, wherein the cross sectional geometry (w₁, h₁) of the vessel (110) comprises a cross-sectional shape of the vessel, or a cross-sectional dimension of the vessel such as a cross-sectional diameter (w₁, h₁) of the vessel, or a cross-sectional area of the vessel.

12. The system according to any previous claim, wherein the orientation (α, β) comprises:
an angle in a two-dimensional coordinate system, or an angle in a three-dimensional coordinate system
and/or
a rotational angle.

13. The system according to any previous claim, wherein the registering (S140) the fluoroscopic image to the one or more angiographic images, is performed based on a shape of the intravascular imaging device (140) in the fluoroscopic image and a shape of the vessel (110) in the one or more angiographic images.

14. A computer-implemented method of determining a cross-sectional geometry of a vessel (110), the method comprising:
receiving (S110) intravascular imaging device data (130) representing a cross sectional geometry (w₁, h₁) of the vessel (110), wherein the intravascular image data (130) is generated by an intravascular imaging device (140) disposed in the vessel (110);
receiving (S120) fluoroscopic data (150), the fluoroscopic data comprising one or more fluoroscopic images representing the intravascular imaging device (140) in the vessel (110) during the generation of the intravascular imaging device data (130);
receiving (S130) angiographic data (160), the angiographic data comprising one or more angiographic images representing the vessel (110); and
for at least one of the one or more fluoroscopic images:
registering (S140) the fluoroscopic image to the one or more angiographic images to determine an orientation (α, β) of the intravascular imaging device (140) with respect to the vessel (110) in the one or more angiographic images;
adjusting (S150) the cross sectional geometry (w₁, h₁) of the vessel (110) based on the orientation (α, β) of the intravascular imaging device (140) with respect to the vessel (110) in the one or more angiographic images; and
outputting (S160) the adjusted cross-sectional geometry (w'₁, h'₁) of the vessel (110).

15. A computer program product comprising instructions which when executed by one or more processors (120), cause the one or more processors to carry out the method according to claim 14.
